# EUROPEAN PATENT SPECIFICATION

(11) **EP 3 089 652 B1**
(45) Date of publication and mention of the grant of the patent: **23.12.2020**
(21) Application number: 14781724.1
(22) Date of filing: 12.09.2014
(51) Int. Cl.: A61B 5/00, A61B 5/04, A61B 5/0245

(54) **METHOD AND SYSTEM FOR DETERMINING THE CLINICAL RELEVANCY OF ALARM EVENTS**
VERFAHREN UND SYSTEM ZUR BESTIMMUNG DER KLINISCHEN RELEVANZ VON ALARMEREIGNISSEN
PROCÉDÉ ET SYSTÈME POUR DÉTERMINER LA PERTINENCE CLINIQUE D'ÉVÉNEMENTS D'ALARME

(30) Priority: 30.12.2013 US 201314143145
(43) Date of publication of application: 09.11.2016
(73) Proprietor: General Electric Company, Schenectady, NY 12345 (US)
(72) Inventor: FULLER, Scott, Milwaukee, Wisconsin 53223 (US); TREACY, Stephen, Milwaukee, Wisconsin 53223 (US)
(74) Representative: Fennell, Gareth Charles
(86) International application number: PCT/US2014/055368
(87) International publication number: WO 2015/102683

(56) References cited:
- WO-A1-2012/140547
- WO-A1-2013/171620
- US-A1- 2009 275 807
- US-A1- 2010 324 377
- US-A1- 2012 154 152
- US-A1- 2012 323 086

## Description

### BACKGROUND OF THE INVENTION

The present disclosure generally relates to physiological monitoring systems. More specifically, the present disclosure relates to a method and system for assessing alarm events that occur in a physiological monitoring system.

Physiological monitoring systems typically include one or more patient monitoring devices that each are able to monitor physiological signals associated with a patient. The patient monitoring devices are used by clinicians in the hospital setting to monitor multiple physiological health parameters of a patient. In addition to providing a real-time display of numerical and waveform measurements, the patient monitoring devices alert clinicians when a monitored signal crosses an alarm threshold setting. The patient monitor generates an alarm event at a priority level that is based upon the criticality of the alarm. The patient monitoring device typically provides the ability to configure the alarm threshold settings and priority levels that are adjusted based upon the criticality of the patient.

This traditional approach to generate alarm events for threshold limit violations creates alarms that are accurate in the sense that the patient monitor reliably generates an alarm when the measured parameter value exceeds or falls below the alarm threshold. However, the simple generation of alarms based upon violations of alarm thresholds does not reliably provide alarms that are deemed actionable by the clinician. The ability to adjust the patient monitor alarm threshold settings and priority levels by the clinician based upon the criticality of the patient is underutilized because of the clinician's inability to analyze the patient's condition and determine what the appropriate configuration should be to increase the utilization of the alarm events.

US-2012/323086-A1, US-2009/275807-A1, US-2010/324377-A1, WO-2012/140547-A1, US-2012/154152-A1, and WO2013/171620-A1 show different alarm management systems.

Increasing the clinical relevancy of a single threshold alarm event requires that the clinician must understand how the most recent alarm event relates to the monitoring history for the patient. If a single alarm event is a transient alarm event that was caused by a motion artifact or a clinical procedure, the alarm event should be characterized as clinically irrelevant. However, if the alarm event is one of many alarm events that have occurred in recent history, the present alarm event may reflect a deterioration in the patient condition. When a clinician is continuously alerted to alarm conditions that, if the clinician is presented with additional information, are deemed to be insignificant and non-actionable, the clinician may begin to ignore the alarm events or to determine the events as lower priority in their workflow. In addition, the non-actionable alarms may be caused by faulty sensors, patient movement, clinical procedures or equipment malfunctions.

Therefore, a need has been recognized to provide metrics that enable clinicians to assess the clinical relevancy of a threshold alarm event by providing the clinician with a clinical relevancy score that is based upon qualitative measures and rules defined by the clinician. Further, a need exists to allow the priority of an alarm event to be escalated based upon the qualitative measures and the clinical relevancy.

### SUMMARY OF THE INVENTION

The invention is defined by the claims.

In an exemplary embodiment of the present disclosure, a method for assessing an alarm event generated in a physiological monitoring system including one or more patient monitors is provided. The method initially validates an alarm event for a monitored patient physiological parameter in a processor. If the alarm event is valid, the method identifies one or more qualitative measures that are associated with the alarm event.

The one or more qualitative measures associated with the alarm event can include one or more of a calculated trajectory of the monitored physiological parameter, a calculated time period between alarm events, and a calculated duration of the current alarm event. Each of these three possible qualitative measures are calculated for the alarm event identified and validated by the processor.

The processor, upon receiving the one or more qualitative measure associated with the alarm event, determines a clinical relevancy score for the alarm event. The clinical relevancy score provides an indication of the relevancy of the alarm event based upon the qualitative measures. Upon determining the clinical relevancy score, the method conveys the clinical relevancy score to a clinician. The clinical relevancy score can be conveyed on a visual display for analysis by the clinician. The clinical relevancy score can be displayed alone or in combination with the alarm event. The clinical relevancy score can further be displayed along with both the alarm event and any of the qualitative measures used to calculate the clinical relevancy score.

In addition to displaying the clinical relevancy score, the method can also escalate the alarm event and, based upon such escalation, generate an urgent alarm to the clinician. The urgent alarm can be generated utilizing any one of several types of alarm devices. The escalation of the alarm event allows the method of the present disclosure to enhance the level of the alarm and more quickly obtain the attention from a clinician.

In another exemplary embodiment of the disclosure, a physiological monitoring system is provided. The system includes one or more patient monitors that are each configured to monitor one or more physiological parameters of a patient. Each of the patient monitors are configured to generate an alarm event when the monitored physiological parameter is outside of alarm thresholds. In addition to the patient monitors, the physiological monitoring system includes a processor that includes a clinical relevancy score module. The clinical relevancy score module of the processor is configured to identify one or more qualitative measures associated with the alarm event. These qualitative measures can include a calculated trajectory of the monitored physiological parameter, a calculated time period between alarm events and a calculated duration of the current alarm event.

Based upon the qualitative measures, the processor can determine a clinical relevancy score for the alarm event. The clinical relevancy score can be used by a clinician to assess the clinical relevancy of the alarm event. The clinical relevancy score can be displayed or otherwise conveyed to a clinician. In addition to displaying or conveying the clinical relevancy score, the physiological monitoring system can also include a display panel that displays each of the qualitative measures along with the alarm event that lead to the clinical relevancy score.

### BRIEF DESCRIPTION OF THE DRAWINGS

The drawings illustrate the best mode presently contemplated of carrying out the disclosure. In the drawings:
Fig. 1 illustrates a physiological monitoring system in accordance with one embodiment of the present disclosure; and
Fig. 2 is a flowchart illustrating a method for calculating a clinical relevancy score in a physiological monitoring system in accordance with the present disclosure.

### DETAILED DESCRIPTION OF THE INVENTION

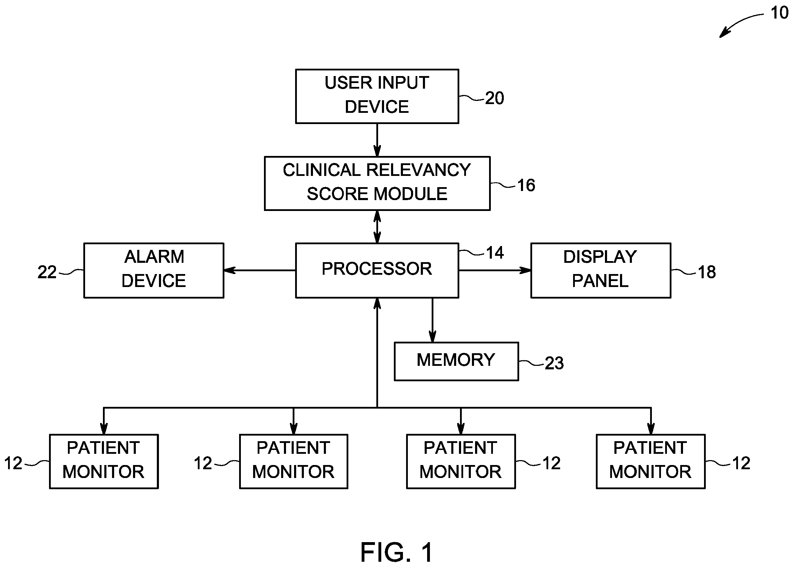

Fig. 1 illustrates a physiological monitoring system 10 in accordance with one embodiment of the present disclosure. The physiological monitoring system 10 includes one or more patient monitors 12, a processor 14, a clinical relevancy score module 16 and a display panel 18. The patient monitors 12 each monitor one or more physiological parameters from a patient associated with the patient monitor 12. Each of the patient monitors 12 may communicate with the processor through either a wired or wireless communication path. Each of the patient monitors 12 is configured to monitor the one or more physiological parameters and generate an alarm signal when any one of the monitored physiological parameter violates an alarm threshold. The alarm threshold can be set in each of the patient monitors 12. Alternatively, the alarm threshold can be set in the processor 14 that communicates with each of the separate patient monitors 12 to receive data related to the monitored physiological parameters.

The one or more alarm events that can be detected by each of the separate patient monitors can include various different physiological conditions of the one or more patients. As an example, the patient monitors 12 can monitor, for example, one or more of an electrocardiogram (ECG), non-invasive blood pressure (NIBP), specific blood oxygen (SpO₂), heart rate, temperature or any other physiological parameter that may be relevant to the overall health of the patient.

As described above, the processor 14 is in communication with each of the patient monitors and is operable to either initially generate and alarm event or validate the accuracy of the one or more alarm events identified by the patient monitor 12 by checking to see if the alarm event falls outside of the alarm thresholds set within the patient monitor. As indicated above, either the patient monitor 12 or the processor 14 can compare the monitored physiological parameter from the patient to the alarm thresholds set in the patient monitor 12 or the processor 14. Various different types of alarm events can be monitored by the patient monitors 12 such as, but not limited to, premature ventricular contraction (PVC), heart rate (HR), temperature, blood pressure (BP) or blood oxygen level.

The clinical relevancy score module 16 is in communication with the processor 14 and is operable to assess the alarm event generated by any one of the patient monitors 12. The clinical relevancy score module 16 utilizes one or more qualitative measures to generate a clinical relevancy score that indicates whether the alarm event being generated by any one of the patient monitors 12 is clinically relevant and should be immediately addressed by the clinician. Likewise, the clinical relevancy score can be used to determine that an alarm event is not clinically relevant and can be given lower priority by the clinician.

The clinical relevancy score module 16 can communicate directly to the display panel 18 while also being able to communicate with the processor 14. A user input device 20 is in communication with the clinical relevancy score module 16 and allows a clinician to input rules and various other metrics that are stored in a memory device 23 that allow the clinical relevancy score module 16 to generate a clinical relevancy score based upon one or more qualitative measures determined by the processor 14. The clinical relevancy score module 16 generates a clinical relevancy score each time an alarm event is generated by one of the patient monitors 12.

Although the clinical relevancy module 16 and the processor 14 are both shown as being separate from each of the individual patient monitors 12, it should be understood that the clinical relevancy score module 16 and the processor 14 could be incorporated into each of the patient monitors 12 such that the patient monitor 12 could operate independently without requiring any connection to separate components or devices. Additionally, the display panel 18 could be incorporated directly into one of the patient monitors 12 to further aid in allowing the patient monitors to operate independently.

As illustrated in Fig. 1, the processor 14 is in communication with an alarm device 22 that is separate from the display panel 18. The alarm device 22 can be one of multiple different types of alarm devices, such as a central monitoring station, an individual pager worn by a nurse or clinician, a mobile telephone carried by the nurse or clinician or any other type of separate device that is able to generate an alarm signal. The processor 14 is able to communicate to the alarm device 22 through either wired or wireless communication techniques such that should one of the alarm events be elevated to a critical status by the clinical relevancy module 16, the processor 14 can communicate to the alarm device 22.

The display panel 18 is configured to display the alarm event received from one of the patient monitors 12. In addition, the display panel 18 can display the individual qualitative measures used by the clinical relevancy score module 16 to calculate the clinical relevancy score as well as the clinical relevancy score generated bv the module 16. In this manner, the staff technician operating the physiological monitoring system 10 can not only view the alarm events but can also view the clinical relevancy score and the individual qualitative measures that were used to develop the clinical relevancy score.

In one embodiment of the disclosure, the physiological monitoring system 10 is part of a centralized patient monitoring system that includes a plurality of patient monitors 12. It will be apparent to those skilled in the art that the physiological monitoring system 10 may also operate as a stand-alone device, such as a bedside patient monitor.

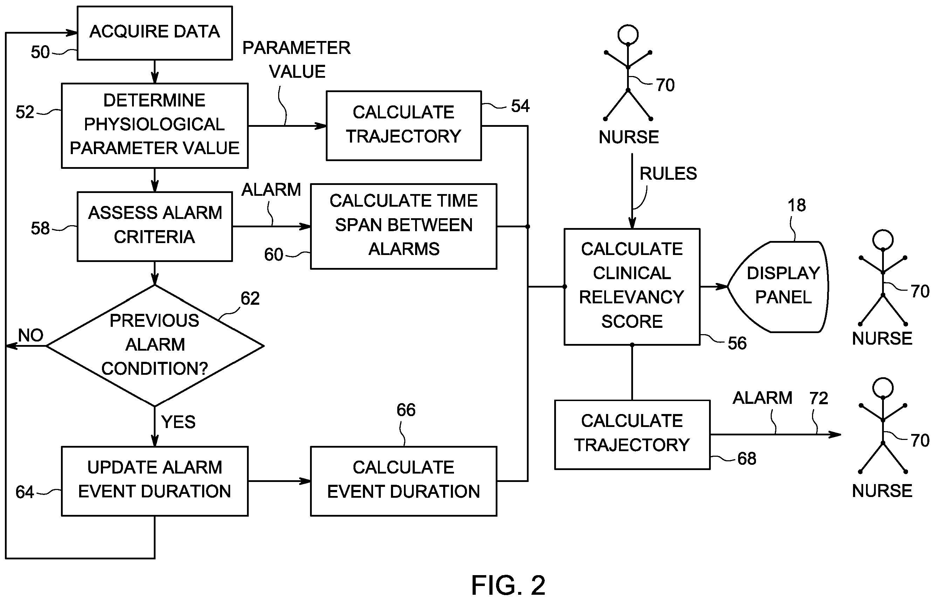

Fig. 2 is a flowchart illustrating a method for assessing an alarm event in the physiological monitoring system shown in Fig. 1. In step 50, the processor 14 initially acquires data from the patient monitor 12 that is monitoring a specific patient. The data acquired in step 50 is the physiological signal obtained directly from the patient monitor for the one or more physiological parameters being monitored by the physiological monitoring system.

Once the processor acquires the data related to the physiological parameter, the system proceeds to step 52 in which the data is used to determine a physiological parameter value. As an example, if the physiological signal is an ECG signal, the processor will process the signal to generate the parameter value being analyzed. In another example, if the physiological signal being monitored is patient temperature, the processor will only need to convert the electronic signal from the patient monitor into a temperature value. After the parameter value is determined, the value is analyzed and compared to values for the physiological parameter obtained in the past. The past values can either be retrieved from the patient monitor or stored within the memory device 23 associated with the processor 14. For example, if the physiological parameter being analyzed is the blood pressure of the patient, the processor will compare the current blood pressure reading to blood pressure readings taken over a period of time to create a trended value for the physiological parameter.

In the example shown, the system may determine in step 52 the overall change in the physiological parameter over a predetermined time period, such as ten minutes. Based upon this historic data, the system calculates a first qualitative measure, which is referred to as the data trajectory. The data trajectory calculated in step 54 is presented as a qualitative measurement. The trajectory assessment carried out in step 54 provides a measure of the strength of the trajectory in the physiological parameter value that is associated with an alarm event. For example, if the blood pressure exceeds an upper alarm threshold, the system performs a trajectory assessment in step 54 and generates a qualitative measure.

As an illustrative example, if the blood pressure has trended consistently with little variation over the measurement period and suddenly has an increase or a decrease that triggers a threshold alarm event, the system calculates a low qualitative measure in step 54. A sudden increase in blood pressure may be due to patient movement, a medical event performed on the patient, or some other type of event that would cause a sudden increase in the monitored physiological parameter. A sudden increase in the monitored parameter is assigned a low qualitative measurement value as described. Alternatively, if the parameter value being monitored, such as blood pressure, has been trending consistently upward for the extended period of time and ultimately results in the parameter exceeding the alarm threshold, the qualitative measure based upon the trajectory, as determined in step 54, is set at a high value. In this manner, step 54 is able to calculate a qualitative measure based upon a trajectory assessment for the physiological parameter being monitored.

In one embodiment of the disclosure, the qualitative measure can have a value between one and five, where five indicates a more relevant alarm, such as the physiological parameter trending consistently upward, while one represents a qualitative measure having a low relevancy, such as an instantaneous spike in blood pressure due to some outside influence. The range of one to five for the qualitative measure is only an illustrative example and the actual values for the range can be adjusted according to design parameters for the system. However, it is contemplated that the same range will be used for all of the qualitative measures and that one end of the range will be for more relevant alarm events while the opposite end of the range will be for less relevant alarm events.

As indicated in Fig. 2, the qualitative measure calculated by step 54 is fed to step 56 in which the system calculates a clinical relevancy score. In the embodiment shown in Fig. 2, multiple qualitative measures, if available, may be used in step 56 to calculate the clinical relevancy score.

Following step 52, the system moves to step 58 in which the system compares the determined physiological parameter value to alarm thresholds that are stored in the memory associated with the processor. If the physiological parameter value violates the alarm threshold, the system determines a second qualitative measure in step 60. The qualitative measure determined in step 60 is based upon the time span between the alarm events related to the monitored physiological parameter. Once again, utilizing blood pressure as the monitored physiological parameter, the system determines in step 60 the amount of time between alarms indicating that the patient's blood pressure has either exceeded an upper alarm threshold or fallen below a lower alarm threshold.

If the system determines in step 60 that the time span between alarms is long, the qualitative measure generated by step 60 will be low. Conversely, if the time span between alarm events is short, indicating that the monitored physiological parameter has fallen outside of the alarm threshold multiple times in a relatively short period of time, the qualitative measure generated by step 60 will be high. As an illustrative example, the qualitative measure determined in step 60 could fall in the range of one to five, where five indicates multiple alarm events that have occurred in a small amount of time while a qualitative measure value of one indicates that the time span between alarm events is relatively long. The qualitative measure calculated in step 60 is provided to step 56 where the system calculates a clinical relevancy score based either solely upon the qualitative measure from step 60 or based on a combination with the qualitative measure from step 54.

Once the time span between alarm events has been determined in steps 58 and 60, the system proceeds to step 62 in which the processor determines whether the alarm condition is a continuing alarm. For example, if the patient's blood pressure has exceeded the upper alarm threshold for a continuous period of time, the system determines that the alarm is a continuing alarm and proceeds to step 64. If the alarm was just triggered and is not a continuing alarm, the system returns to step 50 and continues to acquire data related to the monitored physiological parameters for the patient.

Once the alarm event duration has been updated in step 64, the system proceeds to step 66 in which another qualitative measure is calculated. In step 66, the system calculates a qualitative measure that is based upon the duration of the current alarm event. The qualitative measure value calculated in step 66 will depend upon how long the alarm event has been occurring and the physiological parameter has fallen outside of the alarm parameters. A qualitative value of five will be assigned for an alarm event that has been occurring for an extended period of time while a qualitative measure value of one will be assigned for an alarm event that has been occurring for only a relatively short period of time. The qualitative measure calculated in step 66 is also provided to the processor such that the processor can calculate the clinical relevancy score in step 56.

In step 56, the processor analyzes each of the qualitative measures that are received from steps 54, 60 and 66. In some instances, the processor may only receive one of the qualitative measures, such as if the monitored physiological parameter exceeds the alarm threshold for the first time. In such a case, the time span between alarms, which is used in step 60, would be zero and the duration of the alarm event, calculated in step 66, would also not be available. In such a case, the system would calculate the clinical relevancy score based solely upon the qualitative measure received from the trajectory evaluation in step 54. Conversely, if the alarm event closely follows another recent alarm event and has an extended duration, the system will utilize all three qualitative measures in calculating the clinical relevancy score in step 56. In such a situation, the clinical relevancy score calculated will be much higher and will trigger an alarm escalation, as indicated in step 68.

In the embodiment shown in Fig. 2, a clinician 70, such as a nurse or doctor, can enter a set of operating rules and/or algorithms into the processor for use by the processor in calculating the clinical relevancy score. As an example, the rules entered by the clinician 70 can indicate when the alarm status is escalated and when an alarm is sent to a clinician, such as illustrated by the alarm 72 shown in Fig. 2. Likewise, the rules entered by the clinician 70 can also be used to characterize when an alarm event is clinically irrelevant and can be de-emphasized.

As shown in Fig. 2, once the clinical relevancy score is calculated in step 56, the clinical relevancy score is displayed on the display panel 18. The display panel 18 is viewable by a clinician 70 along with an indication of the actual alarm event that triggered the calculation of the clinical relevancy score. The clinical relevancy score can be displayed to the clinician 70 in many different ways. As an example, various colors, flashing indicators, symbols, position on the screen, location in a listing of alarm events or any other visual display can be used to indicate the clinical relevancy score to the clinician. In addition to displaying the clinical relevancy score with the alarm event, the processor can also display each of the qualitative measures determined in steps 54, 60 and 66. By presenting the alarm event, each of the qualitative measures and the clinical relevancy score to the clinician on the same display panel 18, the clinician is able to analyze the alarm event based upon the determined clinical relevancy score and each of the qualitative measures. Thus, the clinician is able to determine not only the clinical relevancy but also analyze which of the qualitative measures have the highest value and were used to calculate the clinical relevancy score. As an example, if the clinician sees that the alarm has been generated for an excessively long period of time, as determined by the qualitative measure from step 66, this may carry additional significance to the clinician. Likewise, an alarm event that has a high qualitative measure based upon the trajectory assessment carried out in step 54 may also have additional significance to the clinician 70.

As indicated above, if the clinical relevancy score is high enough, the processor can be programed to escalate the alarm event. For example, if the alarm event has high qualitative scores based upon the trajectory of the monitored physiological parameter, the frequency of the alarm events and the duration of the current alarm, the system may determine in step 68 that the alarm event is a critical alarm. If the alarm event is deemed critical, the system may generate an alarm 72 to a hospital information system, a pager carried by the clinician or any other alarm that has the highest priority within the hospital environment.

As indicated in the description above, the method and system of the present disclosure is able to assess the clinical relevancy of a single alarm event and, based upon qualitative measures, calculate a clinical relevancy score. The clinical relevancy score is presented on a display panel along with the single alarm event such that the clinician is able to appreciate the significance and clinical relevancy of the current single alarm event.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A method for assessing an alarm event generated in a physiological monitoring system (10), the method comprising:
validating the alarm event for a monitored physiological parameter in a processor (14);
identifying one or more qualitative measures associated with the alarm event, wherein
the qualitative measures comprise one or more of:
a) a calculated trajectory of the monitored physiological parameter, wherein the calculated trajectory is calculated based upon the overall change in the physiological parameter over a predetermined period of time;
b) a calculated time period between alarm events; and
c) the duration of the alarm event;
wherein each of the one or more qualitative measures comprises a value from a range, wherein one end of the range is for more clinically relevant alarm events and the opposite end of the range is for less clinically relevant alarm events;
determining a clinical relevancy score for the alarm event based on the value of the one or more qualitative measures, wherein the clinical relevancy score indicates the priority that the clinician should give the alarm event; and
displaying the clinical relevancy score and the alarm event.

2. The method of claim 1 further comprising the step of escalating the alarm event and generating an urgent alarm based upon the qualitative measures.

3. The method of claim 1 wherein the clinical relevancy score and each of the one or more qualitative measures are displayed along with the alarm event.

4. The method of claim 1 wherein the clinical relevancy score is based upon a plurality of qualitative measures including a calculated trajectory of the monitored physiological parameter, a calculated time period between alarm events and a calculated duration of the alarm event.

5. A physiological monitoring system (10) comprising:
one or more patient monitors (12) each configured to monitor one or more physiological parameters of a patient and generate an alarm event when the monitored physiological parameter is outside of alarm thresholds; and
a processor (14) including a clinical relevancy score module (16) configured to:
identify one or more qualitative measures associated with the alarm event,
wherein the qualitative measures comprise one or more of:
a) a calculated trajectory of the monitored physiological parameter, wherein the calculated trajectory is calculated based upon the overall change in the physiological parameter over a predetermined period of time;
b) a calculated time period between alarm events; and
c) the duration of the alarm event;
wherein each of the one or more qualitative measures comprises a value from a range, wherein one end of the range is for more clinically relevant alarm events and the opposite end of the range is for less clinically relevant alarm events;
determine a clinical relevancy score for the alarm event based on the value of one or more qualitative measures; and
convey the clinical relevancy score, wherein the clinical relevancy score indicates the priority that the clinician should give the alarm event;
and a display panel configured to display the clinical relevancy score and the alarm event.

6. The physiological monitoring system of claim 5 wherein the display panel is configured to display each of the qualitative measures along with the alarm event.

7. The physiological monitoring system of claim 5 or claim 6 wherein the processor is part of a centralized patient monitoring system located separate from the patient monitors.

8. The physiological monitoring system of any of claims 5 to 7, further comprising an alarm device in communication with the processor such that the processor can generate an urgent alarm to the alarm device based upon the clinical relevancy score.

9. The method of any of claims 1 to 4, wherein the alarm event comprises a single alarm event and wherein a plurality of qualitative measures associated with the alarm event are identified.

## Patentansprüche

1. Verfahren zur Beurteilung eines Alarmereignisses, das in einem physiologischen Überwachungssystem (10) generiert wurde, wobei das Verfahren umfasst:
Validieren des Alarmereignisses für einen überwachten physiologischen Parameter in einem Prozessor (14) ;
Identifizieren von einem oder mehreren qualitativen Maßen, die mit dem Alarmereignis in Zusammenhang stehen, wobei die qualitativen Maße ein oder
mehrere der folgenden umfassen:
a) eine berechnete Bahn des überwachten physiologischen Parameters, wobei die berechnete Bahn basierend auf der Gesamtveränderung des physiologischen Parameters über einen vorbestimmten Zeitraum berechnet wird;
b) einen berechneten Zeitraum zwischen Alarmereignissen; und
c) die Dauer des Alarmereignisses;
wobei jedes von dem einen oder den mehreren qualitativen Maßen einen Wert aus einem Bereich umfasst, wobei ein Ende des Bereichs für klinisch relevantere Alarmereignisse ist, und das entgegengesetzte Ende des Bereichs für weniger klinisch relevante Alarmereignisse ist;
Ermitteln eines Scores der klinischen Relevanz für das Alarmereignis basierend auf dem Wert des einen oder der mehreren qualitativen Maße, wobei der Score der klinischen Relevanz die Priorität angibt, die der Kliniker dem Alarmereignis einräumen sollte; und
Anzeigen des Scores der klinischen Relevanz und des Alarmereignisses.

2. Verfahren nach Anspruch 1, des Weiteren umfassend den Schritt des Eskalierens des Alarmereignisses und Generieren eines dringenden Alarms basierend auf den qualitativen Maßen.

3. Verfahren nach Anspruch 1, wobei der Score der klinischen Relevanz und jedes von dem einen oder den mehreren qualitativen Maßen zusammen mit einem Alarmereignis angezeigt werden.

4. Verfahren nach Anspruch 1, wobei der Score der klinischen Relevanz auf vielen qualitativen Maßen basiert, die eine berechnete Bahn des überwachten physiologischen Parameters, einen berechneten Zeitraum zwischen Alarmereignissen und eine berechnete Dauer des Alarmereignisses einschließen.

5. Physiologisches Überwachungssystem (10), umfassend:
einen oder mehrere Patientenmonitore (12), die jeweils ausgestaltet sind, um einen oder mehrere physiologische Parameter eines Patienten zu überwachen und ein Alarmereignis zu generieren, wenn der überwachte physiologische Parameter außerhalb der Alarmschwellenwerte liegt; und
einen Prozessor (14), der ein Score-Modul der klinischen Relevanz (16) einschließt, das ausgestaltet ist zum:
Identifizieren von einem oder mehreren qualitativen Maßen, die mit dem Alarmereignis in Zusammenhang stehen, wobei die qualitativen Maße ein oder mehrere der folgenden umfassen:
a) eine berechnete Bahn des überwachten physiologischen Parameters, wobei die berechnete Bahn basierend auf der Gesamtveränderung des physiologischen Parameters über einen vorbestimmten Zeitraum berechnet wird;
b) eine berechnete Zeitperiode zwischen Alarmereignissen; und
c) die Dauer des Alarmereignisses;
wobei jedes von dem einen oder den mehreren qualitativen Maßen einen Wert aus einem Bereich umfasst, wobei ein Ende des Bereichs für klinisch relevantere Alarmereignisse ist, und das entgegengesetzte Ende des Bereichs für weniger klinisch relevante Alarmereignisse ist;
Ermitteln eines Scores der klinischen Relevanz für das Alarmereignis basierend auf dem Wert des einen oder der mehreren qualitativen Maße; und
Übermitteln des Scores der klinischen Relevanz, wobei der Score der klinischen Relevanz die Priorität angibt, die der Kliniker dem Alarmereignis einräumen sollte;
und ein Anzeigefeld, das ausgestaltet ist, um den Score der klinischen Relevanz und das Alarmereignis anzuzeigen.

6. Physiologisches Überwachungssystem nach Anspruch 5, wobei das Anzeigefeld ausgestaltet ist, um jedes der qualitativen Maße zusammen mit dem Alarmereignis anzuzeigen.

7. Physiologisches Überwachungssystem nach Anspruch 5 oder Anspruch 6, wobei der Prozessor Teil eines zentralisierten Patientenüberwachungssystems ist, das sich örtlich getrennt von den Patientenmonitoren befindet.

8. Physiologisches Überwachungssystem nach einem der Ansprüche 5 bis 7, des Weiteren umfassend eine Alarmvorrichtung in Kommunikation mit dem Prozessor, so dass der Prozessor basierend auf dem Score der klinischen Relevanz einen dringenden Alarm an die Alarmvorrichtung generieren kann.

9. Verfahren nach einem der Ansprüche 1 bis 4, wobei das Alarmereignis ein einzelnes Alarmereignis umfasst, und wobei viele qualitative Maße identifiziert werden, die im Zusammenhang mit dem Alarm stehen.

## Revendications

1. Procédé pour évaluer un événement d'alarme généré dans un système de surveillance physiologique (10), le procédé comprenant :
la validation de l'événement d'alarme pour un paramètre physiologique surveillé dans un processeur (14) ;
l'identification d'une ou plusieurs mesures qualitatives associées à l'événement d'alarme, les mesures qualitatives comprenant une ou plusieurs des mesures suivantes :
a) une trajectoire calculée du paramètre physiologique surveillé, la trajectoire calculée étant calculée sur la base du changement global du paramètre physiologique sur une période de temps prédéterminée ;
b) une période de temps calculée entre les événements d'alarme ; et
c) la durée de l'événement d'alarme ;
chacune de la ou des mesures qualitatives comprenant une valeur d'une plage, une extrémité de la plage concernant des événements d'alarme plus pertinents sur le plan clinique et l'extrémité opposée de la plage concernant des événements d'alarme moins pertinents sur le plan clinique ;
la détermination d'un score de pertinence clinique pour l'événement d'alarme sur la base de la valeur de la ou des mesures qualitatives, le score de pertinence clinique indiquant la priorité que le clinicien doit accorder à l'événement d'alarme ; et
l'affichage du score de pertinence clinique et l'événement d'alarme.

2. Procédé selon la revendication 1, comprenant en outre l'étape consistant à faire remonter l'événement d'alarme et à générer une alarme urgente sur la base des mesures qualitatives.

3. Procédé selon la revendication 1, le score de pertinence clinique et chacune des mesures qualitatives étant affichés en même temps que l'événement d'alarme.

4. Procédé selon la revendication 1, le score de pertinence clinique étant basé sur une pluralité de mesures qualitatives, comprenant une trajectoire calculée du paramètre physiologique surveillé, une période de temps calculée entre des événements d'alarme et une durée calculée de l'événement d'alarme.

5. Système de surveillance physiologique (10) comprenant :
un ou plusieurs moniteurs de patient (12) configurés chacun pour surveiller un ou plusieurs paramètres physiologiques d'un patient et générer un événement d'alarme lorsque le paramètre physiologique surveillé est en dehors de seuils d'alarme ; et
un processeur (14) comprenant un module de score de pertinence clinique (16) configuré pour :
identifier une ou plusieurs mesures qualitatives associées à l'événement d'alarme, les mesures qualitatives comprenant une ou plusieurs des mesures suivantes :
a) une trajectoire calculée du paramètre physiologique surveillé, la trajectoire calculée étant calculée sur la base du changement global du paramètre physiologique sur une période de temps prédéterminée ;
b) une période de temps calculée entre des événements d'alarme ; et
c) la durée de l'événement d'alarme ;
chacune de la ou des mesures qualitatives comprenant une valeur d'une plage, une extrémité de la plage concernant des événements d'alarme plus pertinents sur le plan clinique et l'extrémité opposée de la plage concernant des événements d'alarme moins pertinents sur le plan clinique ;
déterminer un score de pertinence clinique pour l'événement d'alarme sur la base de la valeur d'une ou plusieurs mesures qualitatives ; et
transmettre le score de pertinence clinique, le score de pertinence clinique indiquant la priorité que le clinicien doit accorder à l'événement d'alarme ;
et un panneau d'affichage configuré pour afficher le score de pertinence clinique et l'événement d'alarme.

6. Système de surveillance physiologique selon la revendication 5, le panneau d'affichage étant configuré pour afficher chacune des mesures qualitatives en même temps que l'événement d'alarme.

7. Système de surveillance physiologique selon la revendication 5 ou la revendication 6, le processeur faisant partie d'un système centralisé de surveillance de patient, situé séparément des moniteurs de patient.

8. Système de surveillance physiologique selon l'une quelconque des revendications 5 à 7, comprenant en outre un dispositif d'alarme en communication avec le processeur de telle sorte que le processeur puisse générer une alarme urgente vers le dispositif d'alarme sur la base du score de pertinence clinique.

9. Procédé selon l'une quelconque des revendications 1 à 4, l'événement d'alarme comprenant un événement d'alarme unique et une pluralité de mesures qualitatives associées à l'événement d'alarme étant identifiées.
